# EUROPEAN PATENT APPLICATION

(11) **EP 4 776 303 A1**
(43) Date of publication of application: **15.07.2026**
(21) Application number: 25151808.0
(22) Date of filing: 14.01.2025
(51) Int. Cl.: G16H 50/30, A61B 5/01, A61B 5/00

(54) **SYSTEM AND METHOD FOR INFECTION PREDICTION**

(71) Applicant: Koninklijke Philips N.V., 5656 AE Eindhoven (NL)
(72) Inventor: FENG, Ting, Eindhoven (NL); SILVA, Ikaro, Eindhoven (NL); DAMIANO, Robert John, 5656 AG Eindhoven (NL); CONROY, Bryan, Eindhoven (NL); MARIANI, Sara, Eindhoven (NL)
(74) Representative: Philips Intellectual Property & Standards

(57) **Abstract**

A system and method for infection prediction. An infection prediction model, trained using a training dataset acquired in a first set of circumstances, and a set of physiological parameters for a subject, acquired in a second, different set of circumstances, are obtained. Data values identified as contextually different to the training dataset are identified in and removed from the set of physiological parameters to generate a modified set of physiological parameters. Input features are generated from the modified set of physiological parameters and provided to the infection prediction model to generate an infection prediction score for the subject.

## Description

This invention was made with government support under Contract No. HDTRA121C0006 awarded by the Unted States Department of Defense (Defense Threat Reduction Agency). The government has certain rights in the invention.

### FIELD OF THE INVENTION

The invention relates to the field of infection prediction, and, in particular, to infection prediction using an infection prediction model.

### BACKGROUND OF THE INVENTION

The COVID-19 pandemic highlighted the importance of early detection of infections, and the need for health monitoring solutions outside clinical settings. Infection prediction models using wearable physiological data have been shown to be capable of predicting infection early (i.e. before the infection would be detected by diagnostic testing). However, training these infection prediction models requires data from a large number of positive cases, which is difficult and time-consuming to obtain. This means that, in the early stages of an outbreak of an infection, when identifying infections is particularly valuable in order to prevent transmissions, these infection prediction models are not available due to a lack of suitable training data.

Data from positive cases may be more easily obtained in clinical settings such as hospitals. Thus, an infection prediction model may be trained in the early stages of or prior to an outbreak using training data acquired in clinical settings. However, an infection prediction model trained using training data acquired in clinical settings provides poor results when used to predict infections outside these clinical settings.

### SUMMARY OF THE INVENTION

The invention is defined by the claims.

According to examples in accordance with an aspect of the invention, there is provided a processing system for infection prediction, the processing system being configured to: obtain a trained infection prediction model configured to output an infection prediction score based on input features generated from a set of physiological parameters, wherein the trained infection prediction model was trained on a training dataset comprising, for each of a plurality of training subjects, a set of input features generated from a set of physiological parameters for the training subject, wherein the sets of physiological parameters used to generate the sets of input features in the training dataset were acquired under a first set of circumstances; obtain a set of physiological parameters for a first subject, wherein the set of physiological parameters for the first subject was acquired under a second set of circumstances, and wherein each physiological parameter in the set of physiological parameters for the first subject corresponds to a physiological parameter in each set of physiological parameters used to generate the sets of input features in the training dataset; process the set of physiological parameters for the first subject to generate a modified set of physiological parameters, wherein the modified set of physiological parameters is generated by: identifying a subset of data values for each physiological parameter in the set of physiological parameters for the first subject as contextually different to the sets of physiological parameters used to generate the sets of input features in the training dataset; and excluding, from the modified set of physiological parameters, the identified subset of data values for each physiological parameter; and process the modified set of physiological parameters to generate input features for the infection prediction model, each input feature corresponding to an input feature in each set of input features in the training dataset; and provide the generated input features to the trained infection prediction model to generate an infection prediction score for the first subject.

The inventors have recognized that an infection prediction model that has been trained using data acquired under a first set of circumstances (such as in a clinical setting) may be used to more accurately predict infection from data acquired under a different set of circumstances (such as by a wearable device outside clinical settings), by modifying the data acquired under the different set of circumstances to more closely resemble the first set of circumstances before providing the modified data to the infection prediction model.

In some examples, the subset of data values for each physiological parameter is identified as contextually different to the sets of physiological parameters used to generate the sets of input features in the training dataset based on an activity performed during acquisition of the data values in the subset.

In some examples, each of the plurality of training subjects was a patient in a clinical setting during acquisition of the set of physiological parameters for the training subject used to generate the set of input features in the training dataset.

In some examples, the subset of data values for each physiological parameter comprises any data values identified as having been acquired for the first subject during a wakeful state of the first subject.

In other words, only data values acquired while the first subject is asleep are provided to the infection prediction model.

This is based on the recognition that patients in clinical settings are generally sedentary. Excluding data values acquired while the first subject is awake means that the modified set of physiological parameters includes only data values that were acquired in a similar context to the training dataset.

In some examples, the subset of data values for each physiological parameter comprises any data values identified as having been acquired for the first subject during non-sedentary activities.

In other words, only data values acquired while the first subject is sedentary are provided to the infection prediction model.

In some examples, the step of generating input features for the infection prediction model comprises: processing the modified set of physiological parameters to generate a plurality of initial features; and for each initial feature, applying a respective transformation to the initial feature to generate an input feature for the infection prediction model.

This has been shown to further improve the results of the infection prediction model when using data acquired under a different set of circumstances to the training dataset, by adjusting for covariate shift (i.e. a dataset shift caused by a difference in probability for the physiological parameters).

In some examples, the transformation for each input feature is a monotonic transformation.

This provides a straightforward way to correct covariate shift, without needing to re-train the infection prediction model.

In some examples, the transformation for each input feature is defined by: obtaining a further dataset comprising, for each of a plurality of further subjects, a set of input features generated from a set of physiological parameters for the further subject, wherein the sets of physiological parameters used to generate the sets of input features in the further dataset were acquired under the second set of circumstances; and for each input feature, defining, as the transformation for the input feature, a transformation that transforms the data values for the input feature in the further dataset such that a similarity between the data distribution for the input feature in the further dataset and the data distribution for the input feature in the training dataset is increased.

In some examples, the number of subjects in the plurality of further subjects is smaller than the number of subjects in the plurality of training subjects.

It has been found that a suitable transformation may be defined with far fewer subjects than would be required to train an infection prediction model.

In some examples, the set of physiological parameters for the first subject was acquired by a wearable device.

A wearable device enables continuous acquisition of the set of physiological parameters over a period of several hours or days.

In some examples, the set of physiological parameters includes one or more of: a heart rate; a respiratory rate; a temperature; and/or a heart rate variability.

In some examples, the infection prediction score is a probability of COVID-19 infection.

In some examples, the trained infection prediction model is a decision tree ensemble algorithm.

Decision tree algorithms are particularly suitable for infection prediction.

According to examples in accordance with another aspect of the invention, there is provided a computer-implemented method for infection prediction, the computer-implemented method comprising: obtaining a trained infection prediction model configured to output an infection prediction score based on input features generated from a set of physiological parameters, wherein the trained infection prediction model was trained on a training dataset comprising, for each of a plurality of training subjects, a set of input features generated from a set of physiological parameters for the training subject, wherein the sets of physiological parameters used to generate the sets of input features in the training dataset were acquired under a first set of circumstances; obtaining a set of physiological parameters for a first subject, wherein the set of physiological parameters for the first subject was acquired under a second set of circumstances, and wherein each physiological parameter in the set of physiological parameters for the first subject corresponds to a physiological parameter in each set of physiological parameters used to generate the sets of input features in the training dataset; processing the set of physiological parameters for the first subject to generate a modified set of physiological parameters, wherein the modified set of physiological parameters is generated by: identifying a subset of data values for each physiological parameter in the set of physiological parameters for the first subject as contextually different to the sets of physiological parameters used to generate the sets of input features in the training dataset; and excluding, from the modified set of physiological parameters, the identified subset of data values for each physiological parameter; and processing the modified set of physiological parameters to generate input features for the infection prediction model, each input feature corresponding to a input feature in each set of input features in the training dataset; and providing the generated input features to the trained infection prediction model to generate an infection prediction score for the first subject.

There is also provided a computer program product comprising computer program code means which, when executed on a computing device having a processing system, cause the processing system to perform all of the steps of the computer-implemented method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for infection prediction, according to an embodiment of the invention;
Fig. 2 illustrates an example transformation for an input feature; and
Fig. 3 illustrates a computer-implemented method for infection prediction, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for infection prediction. An infection prediction model, trained using a training dataset acquired in a first set of circumstances, and a set of physiological parameters for a subject, acquired in a second, different set of circumstances, are obtained. Data values identified as contextually different to the training dataset are identified in and removed from the set of physiological parameters to generate a modified set of physiological parameters. Input features are generated from the modified set of physiological parameters and provided to the infection prediction model to generate an infection prediction score for the subject.

The inventors have recognized that the removal of contextually different data values enables an infection prediction model trained using data acquired from one set of subjects in one environment to provide a more accurate infection prediction score using data acquired from another subject in a different environment. This, for instance, allows an infection prediction model trained using data acquired from hospital patients to be used to detect an infection in a general population, or in a particular group, such as military service members, athletes, teachers or healthcare professionals.

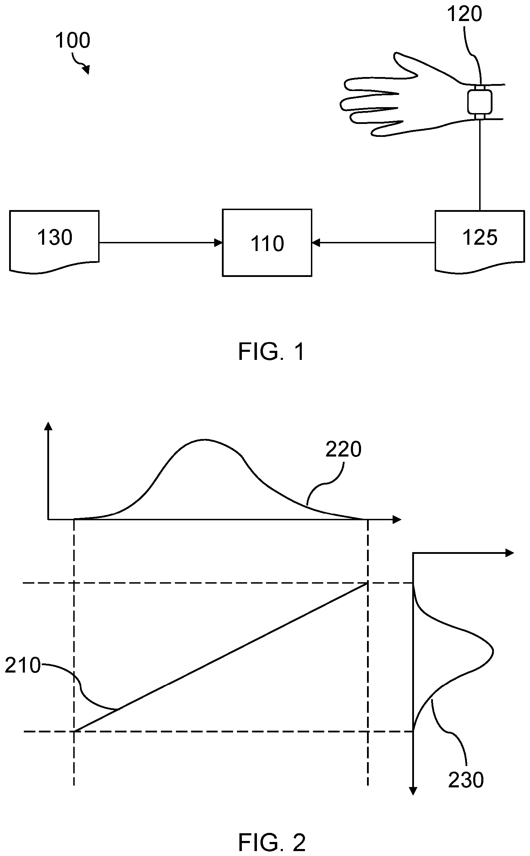

Fig. 1 illustrates a system 100 for infection prediction, according to an embodiment of the invention. The system 100 comprises a processing system 110 and a wearable device 120. The processing system 110 is, itself, an embodiment of the invention.

The processing system 110 is configured to obtain a trained infection prediction model 130. The trained infection prediction model is configured to output an infection prediction score based on input features generated from a set of physiological parameters, and was trained on a training dataset comprising, for each of a plurality of training subjects, a set of input features generated from a set of physiological parameters for the training subject.

The trained infection prediction model may be any machine learning model that has been trained to output an infection prediction score for a subject, i.e. a score indicative of a likelihood that a subject has a given infection, based on an input of features generated from a set of physiological parameters for the subject. For instance, the trained infection prediction model may be a decision tree ensemble algorithm; a decision tree algorithm is particularly suitable for examples in which a feature transformation is performed, as described in more detail below. Further examples of suitable machine-learning algorithms, such as logistic regression, support vector machines and Naive Bayesian models, will be readily apparent to the skilled person. In some examples, a deep learning model may be used; however, in examples in which a feature transformation is performed, the use of deep learning would make defining a suitable transformation more challenging.

The trained infection prediction model may have been trained for any infectious disease. For instance, the infection prediction score output by the trained infection prediction model may be indicative of a likelihood that a subject has COVID-19. In another example, the infection prediction score may be indicative of a likelihood that a subject has influenza. The infection prediction score output by the trained infection prediction model may, for example, be a binary score (e.g. having a value of 0 if the trained infection prediction model predicts that the subject does not have the infection and a value of 1 if the trained infection prediction model predicts that the subject has the infection), or a score between 0 and 1, indicating a probability that the subject has the infection.

One suitable example of a trained infection prediction model is described in Feng et al. (2023), "Machine learning-based clinical decision support for infection risk prediction", Frontiers in Medicine 10.

The sets of physiological parameters used to generate the sets of input features in the training dataset were acquired under a first set of circumstances. The first set of circumstances may be any set of circumstances in which it is possible to determine whether or not a subject has the infection. Preferably the first set of circumstances is a set of circumstances in which a relatively high number of positive cases for the infection or a similar type of infection are found (i.e. compared to a general population), allowing the infection prediction model to be trained at or prior to an early stage of an outbreak of the infection, when the infection may be rare in the general population. For instance, each training subject may have been a patient in a clinical setting (such as a hospital) during acquisition of the set of physiological parameters for the training subject. In clinical settings the acquired set of physiological parameters would often have a higher density (in time) of measured physiological parameter values compared to other settings. This allows each training subject to be tested for the infection, and enables a training dataset in which a relatively high proportion of training subjects test positive for the infection. In other examples, the first set of circumstances may include a (relatively) small and enclosed environment experiencing an outbreak of an infection, in which testing for the infection is available together with the relatively detailed record of physiological parameters change over time. For instance, the training dataset may be acquired from students at a boarding school or passengers on a cruise ship during an outbreak of an infection.

The physiological parameters used to generate the input features in the training dataset are physiological parameters that can be measured (either in the same way or using another method) under different circumstances to the first set of circumstances, under which the physiological parameters for the training dataset were collected. The physiological parameters used to generate the input features in the training dataset used to train the trained infection prediction model may, in some examples, depend on the infection that the trained infection prediction model is trained to predict (i.e. the physiological parameters may be physiological parameters known to be affected by the infection); alternatively, the physiological parameters used to generate the training dataset may be selected without prior knowledge of the infection for which the infection prediction model is trained.

In some examples, each set of physiological parameters may include one or more of: a heart rate, a heart rate variability (for instance, a root mean square of successive differences, RMSSD), a respiratory rate and/or a temperature. The physiological parameters used to generate the input features in the training dataset may be acquired using any suitable technique.

The input features in the training dataset may include, for each physiological parameter in each set, one or more of: a mean, a standard deviation, a maximum and/or a minimum. For instance, the training dataset may include, for each training subject, a mean heart rate, a standard deviation in heart rate, a maximum heart rate, a minimum heart rate, a mean heart rate variability, a standard deviation in heart rate variability, a maximum heart rate variability, a minimum heart rate variability, a mean respiratory rate, a standard deviation in respiratory rate, a maximum respiratory rate, a minimum respiratory rate, a mean temperature, a standard deviation in temperature, a maximum temperature and a minimum temperature. Other suitable input features may additionally or alternatively be included in the training dataset.

The wearable device 120 is configured to acquire a set of physiological parameters 125 for a first subject. Each physiological parameter in the set of physiological parameters for the first subject corresponds to a physiological parameter in each set of physiological parameters used to generate the sets of input features in the training dataset. In Fig. 1, the wearable device 120 is a smartwatch; however, the set of physiological parameters may be acquired by any device or devices capable of acquiring the set of physiological parameters, including other wearable devices, such as a smart ring or a patch, and any suitable non-wearable device.

In some examples, more than one device may be used to acquire the set of physiological parameters for the first subject. For instance, a first subset of physiological parameters for the first subject may be acquired by a first device and a second subset of physiological parameters for the first subject may be acquired by a second device. One or more physiological parameters may belong to both the first subset and the second subset. For example, a smartwatch may be used to measure heart rate and respiratory rate, and a smart ring may be used to measure temperature, heart rate and heart rate variability; the heart rate measurements acquired by the smartwatch may then be combined with the heart rate measurements acquired by the smart ring.

The set of physiological parameters for the first subject is acquired under a second set of circumstances, different to the first set of circumstances. The second set of circumstances may differ from the first set of circumstances with respect to environment. The second set of circumstances may additionally or alternatively differ from the first set of circumstances with respect to population characteristics, such as demographics (e.g. age and/or sex) and/or health state. For instance, the first subject may belong to a demographic that is not represented in the training dataset, or that is underrepresented in the training dataset. The second set of circumstances may additionally or alternatively differ from the first set of circumstances with respect to how the physiological parameters were acquired; for instance, a different type of device may have been used to acquire the physiological parameters and/or a physiological parameter for the first subject may be a proxy for the corresponding physiological parameter in each set of physiological parameters used to generate the sets of input features in the training dataset.

For instance, the training dataset may have been acquired from patients in a clinical setting, while the set of physiological parameters for the first subject may have been acquired in a non-clinical setting. For example, the first subject may belong to the general population, or to a particular profession (e.g. military personnel, athletes, teachers, or healthcare professionals). In these examples, the first subject will typically be younger and healthier than the training subjects. The first subject may have performed activities during acquisition of the set of physiological parameters for the first subject, such as physical exercise and/or travelling, that were not performed by the training subjects (who are typically sedentary) during acquisition of the sets of physiological parameters used to generate the training dataset. The physiological parameters for the first subject may have been acquired in different ways to the corresponding physiological parameter for each training subject; for instance, where the sets of physiological parameters include temperature, this may be core body temperature in the case of the training subjects in the clinical setting, but skin temperature in the case of the first subject. The signal processing algorithms for physiological parameters acquired using clinical equipment in a clinical setting may also differ from the signal processing algorithms for the set of physiological parameters for the first subject; for instance, in a clinical setting, the root mean square of successive differences (RMSSD) may be computed based on pulse estimates of heart beats from an electroencephalogram (EEG) signal, while a photoplethysmography (PPG) signal acquired by the wearable device 120 may be processed to compute RMSSD by detecting fiducial points on the PPG waveforms.

Differences between the first set of circumstances and the second set of circumstances cause a problem known as "dataset shift" in machine learning applications. Dataset shift occurs where the joint distribution of inputs and outputs of a predictive model differ between the training scenario and the testing scenario.

Three types of dataset shift can occur when using the trained infection prediction model, trained using input features generated from physiological parameters acquired under a first set of circumstances, to generate an infection prediction score for the first subject based on input features generated from physiological parameters acquired under a second set of circumstances: covariate shift, label shift and concept drift. Covariate shift occurs when P(X) changes, but P(Y|X) and P(Y) remain the same, where P(X) is the probability distribution of the input features X, P(Y) is the probability distribution of the output infection prediction score Y, and P(Y|X) is the conditional probability distribution of Y given X. Label shift occurs when P(Y) changes, but P(Y|X) and P(X) remain the same. Concept drift occurs when P(Y|X) changes but P(X) and P(Y) remain the same.

The processing system 110 is configured to obtain the set of physiological parameters 125 for the first subject. In Fig. 1, the set of physiological parameters is obtained directly from the wearable device 120 that acquired the set of physiological parameters; however, in other examples, the set of physiological parameters may be obtained from a memory unit (not shown in Fig. 1) storing the set of physiological parameters (or, where relevant, from any another device that acquired the set of physiological parameters).

Having obtained the set of physiological parameters 125 for the first subject, the processing system 110 is configured to process the set of physiological parameters to generate a modified set of physiological parameters. The modified set of physiological parameters is generated by identifying a subset of data values for each physiological parameter in the set of physiological parameters for the first subject as contextually different to the sets of physiological parameters used to generate the sets of input features in the training dataset, and excluding the identified subset of data values for each physiological parameter from the modified set of physiological parameters. In other words, the modified set of physiological parameters contains only data values that have not been identified as contextually different to the sets of physiological parameters used to generate the training dataset.

Contextually different data values are data values acquired under conditions (i.e. conditions likely to affect the value of the physiological parameters) that were not present, or rarely present in the first set of circumstances. The inventors have recognized that excluding these data values, so that they do not affect the output of the trained infection prediction model, reduces both covariate shift and concept drift.

In some examples, the subset of data values for each physiological parameter may be identified as contextually different to the sets of physiological parameters used to generate the sets of input features in the training dataset based on an activity performed during acquisition of data values in the subset.

For instance, if the sets of physiological parameters used to generate the training dataset were acquired from patients in a clinical setting, the data values in the sets of physiological parameters will typically have been acquired while the patient is sedentary. Data values in the set of physiological parameters for the first subject acquired while the first subject is not sedentary may therefore affect the accuracy of an infection prediction score output by the trained infection prediction model; for example, an increase in heart rate due to physical exercise may result in a healthy subject being classified as having an infection by the trained infection prediction model. Thus, the subset of data values for each physiological parameter identified as contextually different may comprise any data values identified as having been acquired for the first subject during a wakeful state of the first subject, so that the modified set of physiological parameters comprises only data values acquired while the first subject is asleep. Alternatively, the subset of data values for each physiological parameter identified as contextually different may comprise any data values identified as having been acquired for the first subject during non-sedentary activities, so that the modified set of physiological parameters comprises only data values acquired while the first subject is sedentary. In some examples, the processing system may additionally or alternatively be configured to identify as contextually different any data values acquired during travel by the first subject and/or during a predetermined period following travel.

In some examples, the processing system 110 may configure the subset of data values for each physiological parameter based on dietary information for the first subject. For instance, if the sets of physiological parameters used to generate the training dataset were acquired from patients in a clinical setting, the processing system may be configured to identify as contextually different any data values acquired during a predetermined period following caffeine intake and/or alcohol intake.

Any other relevant information may be used to identify contextually different data values. For instance, the processing system may be configured to identify as contextually different any data values acquired during a hot bath and/or during a predetermined period following a hot bath.

As the skilled person will appreciate, which contextual differences the processing system 110 is able to identify will depend on the information available to the processing system. For instance, if the device used to acquire the set of parameters is a wearable device that has also acquired hypnogram information for the first subject during the period over which the set of physiological parameters were acquired, the processing system may be configured to receive the hypnogram information acquired by the wearable device, process the hypnogram information to identify sleep segments, and exclude from the modified set of physiological parameters any data values in the set of physiological parameters that were acquired outside the sleep segments. Similarly, if the device used to acquire the set of parameters is a wearable device that has also acquired activity information for the first subject during the period over which the set of physiological parameters were acquired, the processing system may be configured to receive the activity information acquired by the wearable device, process the activity information to identify periods of non-sedentary activity, and exclude from the modified set of physiological parameters any data values in the set of physiological parameters that were acquired during the identified periods of non-sedentary activity. Similarly, if the device used to acquire the set of parameters is a wearable device that is also linked to a cloud application of daily activity logs for the first subject during the period over which the set of physiological parameters were acquired, the processing system may be configured to receive information from the daily activity logs acquired by the cloud application, process the information from the daily activity logs to identify periods of e.g. caffeine intake, hot bath, and travel, and exclude from the modified set of physiological parameters any data values in the set of physiological parameters that were acquired during the identified periods of e.g. caffeine intake, hot bath, and travel.

The processing system 110 is configured to process the modified set of physiological parameters to generate input features for the infection prediction model. Each input feature corresponds to an input feature in each set of input features in the training dataset (for instance, if each set of input features in the training dataset includes a mean heart rate, the generated input features include a mean heart rate generated from the modified set of physiological parameters, and so on).

In some examples, the input features may be generated in a way that accounts for covariate shift (the dataset shift caused by a difference in probability for the physiological parameters), by processing the modified set of physiological parameters to generate a plurality of initial features and, for each initial feature, applying a respective transformation to the initial feature to generate an input feature for the infection prediction model. In other examples, the initial features may be used as the input features that are provided to the infection prediction model (i.e. without applying a transformation).

The transformation for each input feature (i.e. the transformation applied to each initial feature to generate the respective input feature) may be any transformation designed to reduce covariate shift.

In some examples, the transformation may be a monotonic transformation. The inventors have recognized that infection risk as a function of a physiological parameter typically resembles a U-shaped curve, as lower and/or higher values are generally associated with a decline in health. Therefore, a monotonic transformation may be used to modify the shape of the data distribution while preserving the rank order of infection risk.

The transformation for each input feature may be defined using a further dataset comprising, for each of a plurality of subjects, a set of input features generated from a set of physiological parameters for the further subject. The sets of physiological parameters for the further subjects were acquired under the second set of circumstances (i.e. each further subject may belong to the same population as the first subject), and each physiological parameter in the set of physiological parameters for each further subject corresponds to a physiological parameter in each set of physiological parameters used to generate the sets of input features in the training dataset. Each input feature in each set of input features in the further dataset corresponds to an input feature in each set of input features in the training dataset. In other words, the further dataset contains the same types of input feature as the training dataset. The number of subjects in the plurality of further subjects may be smaller than the number of subjects in the plurality of training subjects, and may include proportionally fewer positive cases or zero positive cases. For example, while a robust infection prediction model may be trained using data from thousands of patients, a further dataset of wearable physiology data from 20-30 healthy subjects (e.g. 25 healthy subjects) over a period of, for example, 10-20 days (e.g. a period of 2 weeks) is sufficient to ensure successful generalization of the infection prediction model.

The processing system 110 may be configured to obtain the further dataset, and to define, for each input feature, a transformation that transforms the data values for the input feature in the further dataset such that a similarity between the data distribution for the input feature in the further dataset and the data distribution for the input feature in the training dataset is increased. The transformation defined for each input feature may then be used to generate the corresponding input feature for the first subject (by transforming the corresponding initial feature generated from the modified set of physiological parameters). Alternatively, the processing system may apply a predetermined transformation to each initial feature. The predetermined transformation for each input feature may have been determined using a further dataset as described here.

The transformation that transforms the data values for the input feature in the further dataset such that a similarity between the data distribution for the input feature in the further dataset and the data distribution for the input feature in the training dataset is increased may, for example, be a monotonic transformation defined by matching data rankings (e.g. quantiles). For instance, percentile values may be determined for each input feature in the training dataset and further dataset, and, for each input feature in the further dataset, the values may be replaced with the values for the corresponding input feature in the training dataset that share the same percentile.

Fig. 2 illustrates an example transformation 210 for an input feature. The example transformation is a monotonic transformation defined by matching data rankings that transforms the data distribution 220 of the input feature to a transformed distribution 230, which matches the distribution of the corresponding input feature in the training dataset.

In other examples, the transformation may comprise a non-linear mapping of percentile values. For instance, when the input feature in the training dataset is categorical and the input feature in the further dataset is continuous, the percentile values for an input feature in the training dataset may be mapped to the percentile values for the corresponding input feature in the further dataset using a step function,.

Another example of a suitable transformation is a transformation that matches distributions based on parametric assumptions. For instance, if the input feature in the training dataset is normally distributed, a power transformation (such as a Box Cox transformation) may be applied to the corresponding input feature in the further dataset to convert the corresponding input feature to a normal distribution, and the distribution may then be centered and scaled to match the distribution for the input feature in the training dataset.

In another example, the transformation may be defined by using gradient descent to optimize a transformation function to minimize the Kullback-Leibler divergence between the input feature in the further dataset and the corresponding input feature in the training dataset. A monotonic constraint may be applied to the optimization process.

In yet another example, the transformation may be defined based on a hypothesis determined using the differences between the first set of circumstances and the second set of circumstances (e.g. demographic differences between the training subject population and the first subject). This enables a transformation to be defined without requiring a further dataset.

Having generated the input features, the processing system 110 is configured to provide the generated input features to the trained prediction model 130 to generate an infection prediction score for the first subject. The removal of contextually different data values has been found to improve the performance of the trained infection prediction model in generating an infection prediction score for the first subject, while the transformation of the input features provided to the trained infection prediction model further improves the performance.

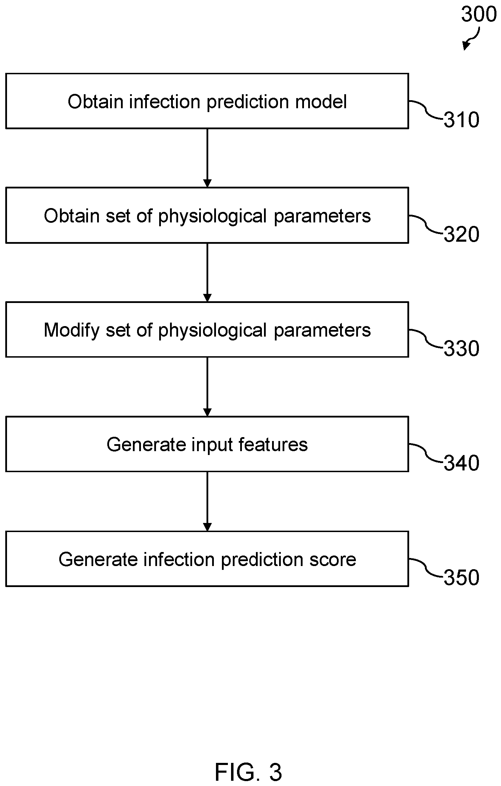

Fig. 3 illustrates a computer-implemented method 300 for infection prediction, according to an embodiment of the invention.

The computer-implemented method 300 begins at step 310, at which a trained infection prediction model is obtained. The trained infection prediction model is configured to output an infection prediction score based on input features generated from a set of physiological parameters, and was trained on a training dataset comprising, for each of a plurality of training subjects, a set of input features generated from a set of physiological parameters for the training subject. The sets of physiological parameters used to generate the sets of input features in the training dataset were acquired under a first set of circumstances.

At step 320, a set of physiological parameters for a first subject is obtained. The set of physiological parameters for the first subject was acquired under a second set of physiological parameters, and each physiological parameter in the set of physiological parameters for the first subject corresponds to a physiological parameter in each set of physiological parameters used to generate the sets of input features in the training dataset.

At step 330, the set of physiological parameters is processed to generate a modified set of physiological parameters. The modified set of physiological parameters is generated by identifying a subset of data values for each physiological parameter in the set of physiological parameters for the first subject as contextually different to the training dataset, and excluding, from the modified set of physiological parameters, the identified subset of data values for each physiological parameter.

At step 340, the modified set of physiological parameters is processed to generate input features for the infection prediction model. In some examples, the input features are generated by processing the modified set of physiological parameters to generate a plurality of initial features, and applying a respective transformation to each initial feature.

At step 350, the generated input features are provided to the trained infection prediction model to generate an infection prediction score for the first subject.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (110) for infection prediction, the processing system being configured to:
obtain a trained infection prediction model (130) configured to output an infection prediction score based on input features generated from a set of physiological parameters, wherein the trained infection prediction model was trained on a training dataset comprising, for each of a plurality of training subjects, a set of input features generated from a set of physiological parameters for the training subject, wherein the sets of physiological parameters used to generate the sets of input features in the training dataset were acquired under a first set of circumstances;
obtain a set of physiological parameters (125) for a first subject, wherein the set of physiological parameters for the first subject was acquired under a second set of circumstances, and wherein each physiological parameter in the set of physiological parameters for the first subject corresponds to a physiological parameter in each set of physiological parameters used to generate the sets of input features in the training dataset;
process the set of physiological parameters for the first subject to generate a modified set of physiological parameters, wherein the modified set of physiological parameters is generated by:
identifying a subset of data values for each physiological parameter in the set of physiological parameters for the first subject as contextually different to the sets of physiological parameters used to generate the sets of input features in the training dataset; and
excluding, from the modified set of physiological parameters, the identified subset of data values for each physiological parameter; and
process the modified set of physiological parameters to generate input features for the infection prediction model, each input feature corresponding to an input feature in each set of input features in the training dataset; and
provide the generated input features to the trained infection prediction model to generate an infection prediction score for the first subject.

2. The processing system (110) of claim 1, wherein the subset of data values for each physiological parameter (125) is identified as contextually different to the sets of physiological parameters used to generate the sets of input features in the training dataset based on an activity performed during acquisition of the data values in the subset.

3. The processing system (110) of claim 2, wherein each of the plurality of training subjects was a patient in a clinical setting during acquisition of the set of physiological parameters for the training subject used to generate the set of input features in the training dataset.

4. The processing system (110) of claim 3, wherein the subset of data values for each physiological parameter comprises any data values identified as having been acquired for the first subject during a wakeful state of the first subject.

5. The processing system (110) of claim 3, wherein the subset of data values for each physiological parameter comprises any data values identified as having been acquired for the first subject during non-sedentary activities.

6. The processing system (110) of any of claims 1 to 5, wherein the step of generating input features for the infection prediction model comprises:
processing the modified set of physiological parameters to generate a plurality of initial features; and
for each initial feature, applying a respective transformation (210) to the initial feature to generate an input feature for the infection prediction model.

7. The processing system (110) of claim 6, wherein the transformation (210) for each input feature is a monotonic transformation.

8. The processing system (110) of claim 6 or 7, wherein the transformation (210) for each input feature is defined by:
obtaining a further dataset comprising, for each of a plurality of further subjects, a set of input features generated from a set of physiological parameters for the further subject, wherein the sets of physiological parameters used to generate the sets of input features in the further dataset were acquired under the second set of circumstances; and
for each input feature, defining, as the transformation for the input feature, a transformation that transforms the data values for the input feature in the further dataset such that a similarity between the data distribution for the input feature in the further dataset and the data distribution for the input feature in the training dataset is increased.

9. The processing system (110) of claim 8, wherein the number of subjects in the plurality of further subjects is smaller than the number of subjects in the plurality of training subjects.

10. The processing system (110) of any of claims 1 to 9, wherein the set of physiological parameters (125) for the first subject was acquired by a wearable device (120).

11. The processing system (100) of any of claims 1 to 10, wherein the set of physiological parameters (125) includes one or more of: a heart rate; a respiratory rate; a temperature; and/or a heart rate variability.

12. The processing system (110) of any of claims 1 to 11, wherein the infection prediction score is a probability of COVID-19 infection.

13. The processing system (110) of any of claims 1 to 12, wherein the trained infection prediction model (130) is a decision tree ensemble algorithm.

14. A computer-implemented method (300) for infection prediction, the computer-implemented method comprising:
obtaining a trained infection prediction model (130) configured to output an infection prediction score based on input features generated from a set of physiological parameters, wherein the trained infection prediction model was trained on a training dataset comprising, for each of a plurality of training subjects, a set of input features generated from a set of physiological parameters for the training subject, wherein the sets of physiological parameters used to generate the sets of input features in the training dataset were acquired under a first set of circumstances;
obtaining a set of physiological parameters (125) for a first subject, wherein the set of physiological parameters for the first subject was acquired under a second set of circumstances, and wherein each physiological parameter in the set of physiological parameters for the first subject corresponds to a physiological parameter in each set of physiological parameters used to generate the sets of input features in the training dataset;
processing the set of physiological parameters for the first subject to generate a modified set of physiological parameters, wherein the modified set of physiological parameters is generated by:
identifying a subset of data values for each physiological parameter in the set of physiological parameters for the first subject as contextually different to the sets of physiological parameters used to generate the sets of input features in the training dataset; and
excluding, from the modified set of physiological parameters, the identified subset of data values for each physiological parameter; and
processing the modified set of physiological parameters to generate input features for the infection prediction model, each input feature corresponding to a input feature in each set of input features in the training dataset; and
providing the generated input features to the trained infection prediction model to generate an infection prediction score for the first subject.

15. A computer program product comprising computer program code means which, when executed on a computing device having a processing system, cause the processing system to perform all of the steps of the computer-implemented method (300) according to claim 14.
